# EUROPEAN PATENT APPLICATION

(11) **EP 2 296 003 A2**
(43) Date of publication of application: **16.03.2011**
(21) Application number: 10171826.0
(22) Date of filing: 04.08.2010
(51) Int. Cl.: G01S 7/52, G01S 15/89

(54) **Providing a three-dimensional ultrasound image based on an ellipsoidal region of interest in an ultrasound system**

(30) Priority: 08.09.2009 KR 20090084254
(71) Applicant: MEDISON CO., LTD., Kangwon-do 250-870 (KR)
(72) Inventor: Kim, Sung Hee, Seoul 135-851 (KR); Kim, Jung, Seoul 135-851 (KR)
(74) Representative: Schmid, Wolfgang

(57) **Abstract**

Embodiments for providing a 3D (three-dimensional) ultrasound image based on an ellipsoidal region of interest (ROI) are disclosed. In one embodiment, by way of nonlimiting example, an ultrasound system comprises: an ultrasound data acquisition unit configured to transmit and receive ultrasound signals to and from a target object to thereby output a plurality of ultrasound data; a user input unit configured to receive input information; and a processing unit in communication with the ultrasound data acquisition unit and the user input unit, the processing unit being configured to form volume data based on the plurality of ultrasound data, set an ellipsoidal region of interest (ROI) on the volume data based on the input information and render volume data corresponding to the ellipsoidal ROI to thereby form a three-dimensional (3D) ultrasound image.

## Description

### CROSS-REFERENCE TO RELATED APPLICATIONS

The present application claims priority from Korean Patent Application No. 10-2009-0084254 filed on September 8, 2009, the entire subject matter of which is incorporated herein by reference.

### TECHNICAL FIELD

The present disclosure generally relates to ultrasound systems, and more particularly to providing a 3D (three-dimensional) ultrasound image based on an ellipsoidal region of interest (ROI) in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modem high-performance ultrasound systems and techniques are commonly used to produce two or three-dimensional diagnostic images of internal features of an object (e.g., human organs).

The ultrasound system may provide a 3D (three-dimensional) ultrasound image including clinical information such as spatial information and anatomical figures of a target object, which cannot be provided through a 2D (two-dimensional) ultrasound image. The ultrasound system may transmit and receive ultrasound signals to and from the target object to thereby form volume data. The ultrasound system may further set a region of interest (ROI) for obtaining the 3D ultrasound image on the volume data. The ultrasound system may further render volume data corresponding to the ROI to thereby form the 3D ultrasound image.

Generally, the ROI has been limited to the shape of a hexahedron. When the ROI is set on the volume data to obtain the 3D ultrasound image corresponding to an object of interest (e.g., kidney, gall, prostate, ovary, etc.) within the target object, the ROI includes the object of interest as well as unnecessary regions. Thus, it is a problem in that the 3D ultrasound image corresponding to the object of interest cannot only be formed.

### SUMMARY

Embodiments for providing a plurality of slice images in an ultrasound system are disclosed herein. In one embodiment, by way of non-limiting example, an ultrasound system comprises: an ultrasound data acquisition unit configured to transmit and receive ultrasound signals to and from a target object to thereby output a plurality of ultrasound data; a user input unit configured to receive input information; and a processing unit in communication with the ultrasound data acquisition unit and the user input unit, the processing unit being configured to form volume data based on the plurality of ultrasound data, set an ellipsoidal region of interest (ROI) on the volume data based on the input information and render volume data corresponding to the ellipsoidal ROI to thereby form a three-dimensional (3D) ultrasound image.

In another embodiment, there is provided a method of providing a 3D ultrasound image, comprising: a) acquiring a plurality of ultrasound data for a target object; b) forming volume data based on the plurality of ultrasound data; c) setting an elliptical region of interest (ROI) on the volume data based on input information from a user; d) setting an ellipsoidal ROI on the volume data based on the elliptical ROI; and e) rendering volume data corresponding to the ellipsoidal ROI to thereby form a 3D ultrasound image.

The Summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

- FIG. 1: is a block diagram showing an illustrative embodiment of an ultrasound system.
- FIG. 2: is a block diagram showing an illustrative embodiment of an ultrasound data acquisition unit.
- FIG. 3: is a schematic diagram showing an example of acquiring ultrasound data corresponding to a plurality of frames.
- FIG. 4: is a block diagram showing an illustrative embodiment of a processing unit.
- FIG. 5: is a flow chart showing a process of forming a 3D (three-dimensional) ultrasound image.
- FIG. 6: is a schematic diagram showing an example of volume data.
- FIG. 7: is a schematic diagram showing an example of a reference plane image and an elliptical region of interest (ROI).
- FIG. 8: is a schematic diagram showing an example of an ellipsoidal ROI.
- FIG. 9: is a schematic diagram showing an example of an YZ plane image and an XZ plane image, which are displayed with the reference plane image.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

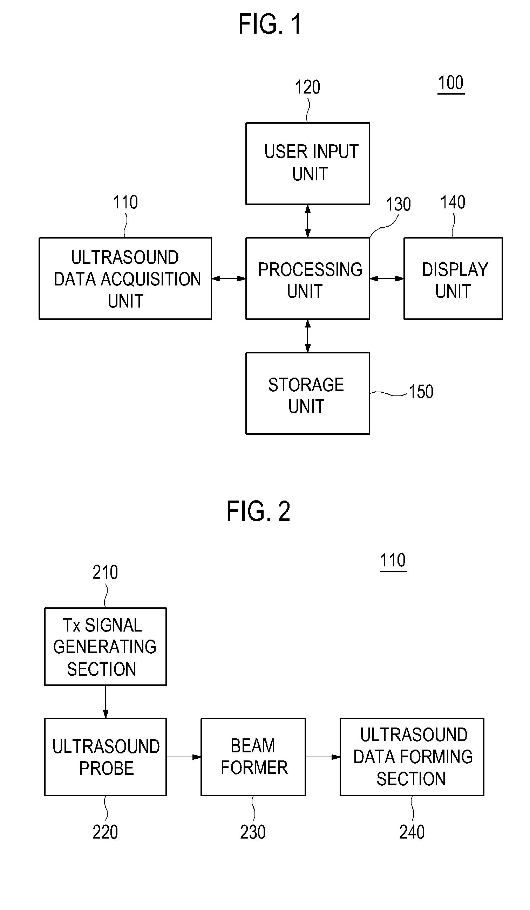

Referring to FIG. 1, an ultrasound system 100 in accordance with an illustrative embodiment is shown. As depicted therein, the ultrasound system 100 may include an ultrasound data acquisition unit 110. The ultrasound data acquisition unit 110 may be configured to transmit and receive ultrasound signals to and from a target object to thereby output ultrasound data.

FIG. 2 is a block diagram showing an illustrative embodiment of the ultrasound data acquisition unit. Referring to FIG. 2, the ultrasound data acquisition unit 110 may include a transmit (Tx) signal generating section 210, an ultrasound probe 220, a beam former 230 and an ultrasound data forming section 240.

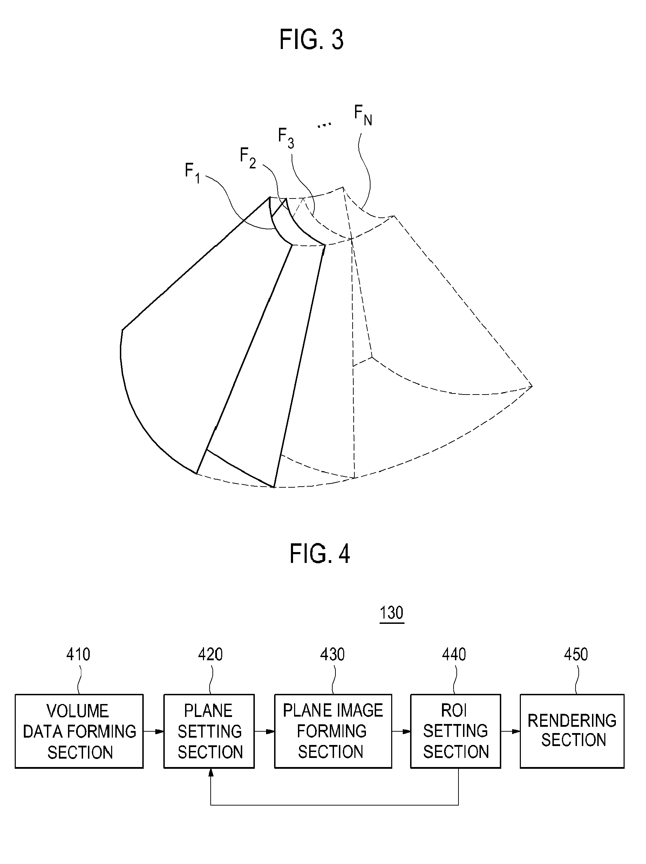

The Tx signal generating section 210 may be configured to generate Tx signals. The Tx signal generating section 210 may generate the Tx signals at every predetermined time to thereby form a plurality of Tx signals for obtaining a plurality of frames Fᵢ (1≤ i ≤ N) representing the target object, as shown in FIG. 3. The frame may include a brightness mode (B mode) image. However, it should be noted herein that the frame may not be limited thereto.

FIG. 3 is a schematic diagram showing an example of acquiring ultrasound data corresponding to the plurality of frames Fᵢ (1≤ i ≤ N). The plurality of frames Fᵢ (1≤ i ≤ N) may represent sectional planes of the target object (not shown).

Referring back to FIG. 2, the ultrasound probe 220 may include a plurality of elements (not shown) for reciprocally converting between ultrasound signals and electrical signals. The ultrasound probe 220 may be configured to transmit ultrasound signals into the target object in response to the Tx signals provided from the Tx signal generating section 210. The ultrasound probe 220 may further receive ultrasound echo signals reflected from the target object to thereby output received signals. The received signals may be analog signals. The ultrasound probe 220 may include a 3D (three-dimensional) mechanical probe, a 2D (two-dimensional) array probe and the like. However, it should be noted herein that the ultrasound probe 220 may not be limited thereto.

The beam former 230 may be configured to convert the received signals provided from the ultrasound probe 220 into digital signals. The beam former 230 may further apply delays to the digital signals in consideration of distances between the elements and focal points to thereby output digital receive-focused signals.

The ultrasound data forming section 240 may be configured to form ultrasound data corresponding to each of the frames Fᵢ (1≤ i ≤ N) based on the digital receive-focused signals provided from the beam former 230. The ultrasound data forming section 240 may further perform various signal processing (e.g., gain adjustment) to the digital receive-focused signals.

Referring back to FIG. 1, the ultrasound system 100 may further include a user input unit 120. The user input unit 120 may be configured to receive input information from a user. In one embodiment, the input information may include first input information for setting a reference plane, second input information for setting an elliptical region of interest (ROI), and third input information for adjusting size of an ellipsoidal ROI. The user input unit 120 may include a control panel, a mouse, a keyboard and the like. However, it should be noted herein that the user input unit 120 may not be limited thereto.

The ultrasound system 100 may further include a processing unit 130. FIG. 4 is a schematic diagram showing an illustrative embodiment of the processing unit. Referring to FIG. 4, the processing unit 130 may include a volume data forming section 410, a plane setting section 420, a plane image forming section 430, an ROI setting section 440 and a rendering section 450.

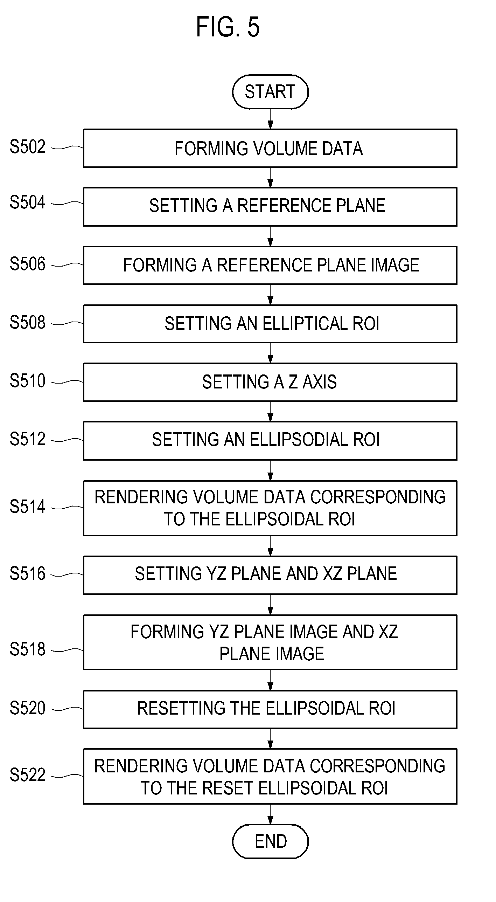

FIG. 5 is a flow chart showing a process of forming a 3D ultrasound image. Referring to FIG. 5, the volume data forming section 410 as shown in FIG. 4 may be configured to form volume data based on the plurality of ultrasound data provided from the ultrasound data acquisition unit 110 as shown in FIG. 1, at step S502.

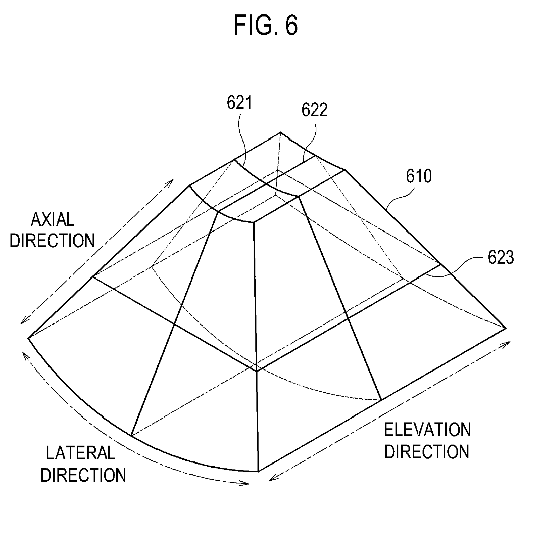

FIG. 6 is a schematic diagram showing an example of the volume data. The volume data 610 may include a plurality of voxels (not shown) having brightness values. In FIG. 6, reference numerals 621 to 623 represent an A plane, a B plane and a C plane. The A plane 621, the B plane 622 and the C plane 623 may be mutually orthogonal. Also, in FIG. 6, the axial direction may be a Tx direction of the ultrasound signals, the lateral direction may be a longitudinal direction of the elements, and the elevation direction may be a swing direction of the elements, i.e., a depth direction of a 3D ultrasound image.

Referring back to FIG. 5, the plane setting section 420 as shown in FIG. 4 may be configured to set the reference plane on the volume data based on the input information (i.e., first input information) provided from the user input unit 120 as shown in FIG. 1, at step S504. The reference plane may be the A plane, the B plane or the C plane. However, it should be noted herein that the reference plane may not be limited thereto.

The plane image forming section 430 as shown in FIG. 4 may be configured to form a reference plane image corresponding to the reference plane based on the volume data, at step S506. The reference plane image may be the B mode image. However, it should be noted herein that the reference plane image may not be limited thereto. The reference plane image may be displayed on a display unit 140 as shown in FIG. 1. Thus, a user may set the elliptical ROI on the reference plane image displayed on the display unit 140.

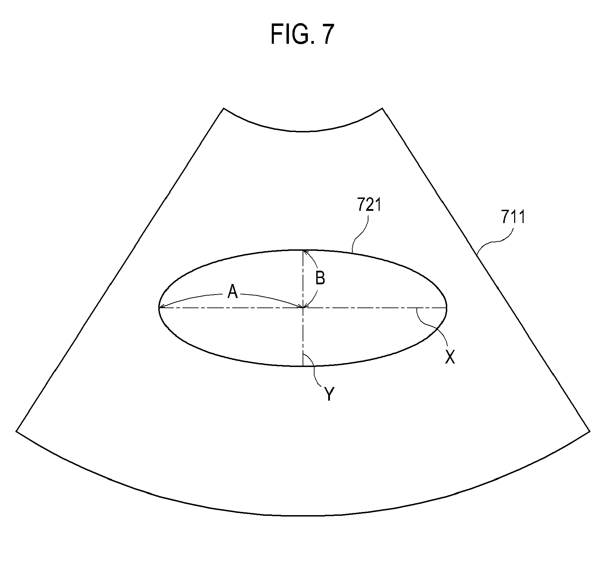

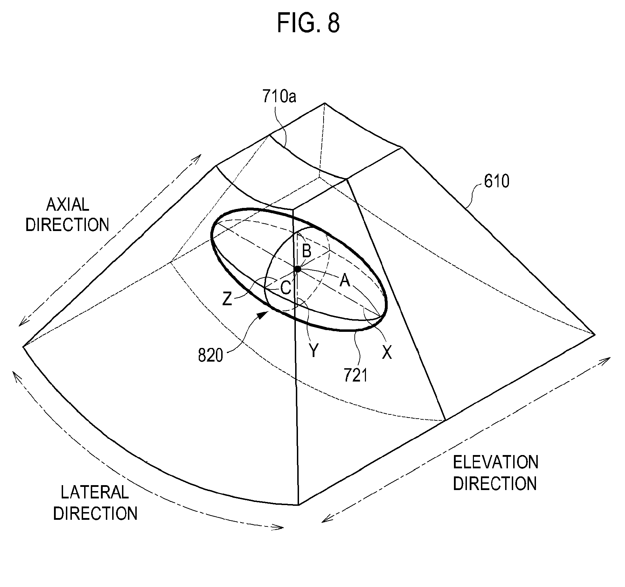

The ROI setting section 440 as shown in FIG. 4 may be configured to set the elliptical ROI on the reference plane of the volume data based on the input information (i.e., second input information) provided from the user input information 120 as shown in FIG. 1, at step S508. FIG. 7 is a schematic diagram showing an example of the reference plane image and the elliptical ROI. As one example, when the input information (i.e., second input information) for setting the elliptical ROI 721 on the reference plane image 711 as shown in FIG. 7 is provided from the user input unit 120 as shown in FIG. 1, the ROI setting section 440 as shown in FIG. 4 may set the elliptical ROI 721 on the reference plane 710a of the volume data 610 as shown in FIG. 8. The elliptical ROI 721 may have an X axis having a radius A and a Y axis Y having a radius B, as shown in FIG. 7. The X axis and the Y axis may be mutually orthogonal.

The ROI setting section 440 as shown in FIG. 4 may set a Z axis that is orthogonal to the X axis and the Y axis on the volume data, at step S510. The ROI setting section 440 may set the ellipsoidal ROI 820 on the volume data 610 based on the elliptical ROI 721 and the Z axis having the radius C as shown in FIG. 8, at step S512.

The rendering section 450 as shown in FIG. 4 may be configured to render volume data corresponding to the ellipsoidal ROI to thereby form the 3D ultrasound image corresponding to the ellipsoidal ROI 820, at step S514. The rendering may include a ray casting rendering, a surface rendering and the like.

The plane setting section 420 as shown in FIG. 4 may further set an YZ plane corresponding to the Y axis and the Z axis of the ellipsoidal ROI 820 and an XZ plane corresponding to the X axis and the Z axis of the ellipsoidal ROI 820 on the volume data 610, at step S516. The YZ plane and the XZ plane may be orthogonal to the reference plane.

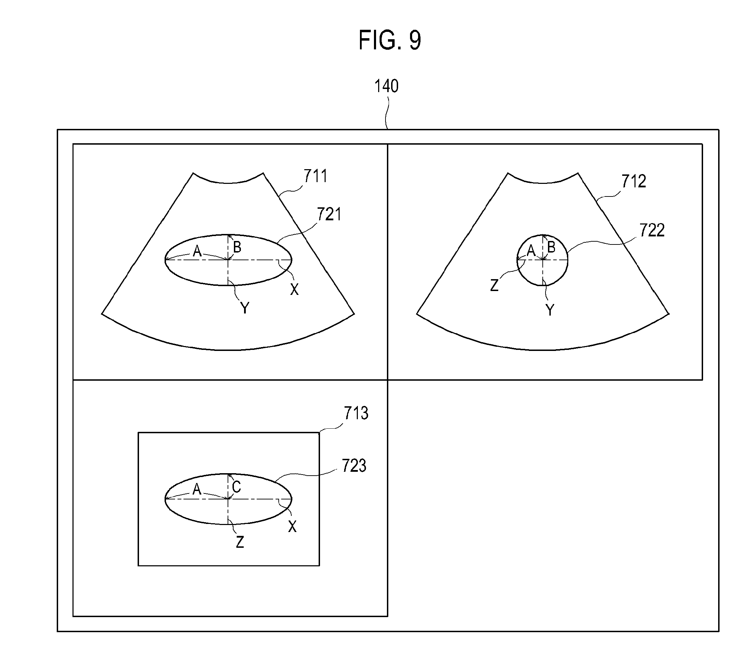

The plane image forming section 430 as shown in FIG. 4 may further form a plane image ("an YZ plane image") 712 corresponding to the YZ plane and a plane image ("an XZ plane image") 713 corresponding to the XZ plane, as shown in FIG. 9, based on the volume data 610 as shown in FIG. 8, at step S518. The YZ plane image 712 and the XZ plane image 713 may include the B mode image. However, it should be noted herein that the YZ plane image 712 and the XZ plane image 713 may not be limited thereto. The YZ plane image 712 and the XZ plane image 713 may be displayed on the display unit 140 with the reference plane image 711, as shown in FIG. 9. Thus, the user may adjust the sizes of elliptical ROIs 721, 722 and 723 corresponding to the reference plane image 711, the YZ plane image 712 and the XZ plane image 713, respectively, as shown in FIG. 9 to thereby adjust the size of the ellipsoidal ROI 820 set on the volume data 610, as shown in FIG. 8.

The ROI setting section 440 as shown in FIG. 4 may further reset the ellipsoidal ROI 820 set on the volume data 610 based on the input information (i.e., the third input information) provided from the user input unit 120 as shown in FIG. 1, at step S520. As one example, when the third input information for adjusting the sizes of elliptical ROIs 721, 722 and 723 corresponding to the reference plane image 711, the YZ plane image 712 and the XZ plane image 713, respectively, as shown in FIG. 9 is provided from the user input unit 120, the ROI setting section 440 may adjust the size of the ellipsoidal RIO 820 set on the volume data 610 to thereby reset the ellipsoidal RIO 820.

The rendering section 450 as shown in FIG. 4 may further render volume data corresponding to the reset ellipsoidal ROI to thereby form the 3D ultrasound image corresponding to the reset ellipsoidal ROI, at step S522.

Referring back to FIG. 1, the ultrasound system 100 may further include the display unit 140. The display unit 140 may display the reference image 711 as shown in FIG. 7. The display unit 140 may further display the YZ plane image 712 and the XZ plane image 713 with the reference image 711, as shown in FIG. 9. The display unit 140 may further display the 3D ultrasound image (not shown).

The ultrasound system 100 may further include a storage unit 150. The storage unit 150 may store the volume data formed by the processing unit 130. The storage unit 150 may include a random access memory (RAM), a hard disk drive, a flash memory and the like.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit configured to transmit and receive ultrasound signals to and from a target object to output a plurality of ultrasound data;
a user input unit configured to receive input information; and
a processing unit in communication with the ultrasound data acquisition unit and the user input unit, the processing unit being configured to form volume data based on the plurality of ultrasound data, set an ellipsoidal region of interest (ROI) on the volume data based on the input information and render volume data corresponding to the ellipsoidal ROI to thereby form a three-dimensional (3D) ultrasound image.

2. The ultrasound system of Claim 1, wherein the processing unit comprises:
a volume data forming section configured to form the volume data based on the plurality of ultrasound data;
a plane setting section configured to set a reference plane on the volume data based on the input information;
a plane image forming section configured to form a reference plane image corresponding to the reference plane based on the volume data;
an ROI setting section configured to set an elliptical ROI on the reference plane of the volume data based on the input information for setting the elliptical ROI on the reference plane image, and set the ellipsoidal ROI on the volume data based on the elliptical ROI; and
a rendering section configured to render volume data corresponding to the ellipsoidal ROI to thereby form the 3D ultrasound image.

3. The ultrasound system of Claim 2, wherein the input information comprises:
first input information for setting a reference plane on the volume data; and
second input information for setting an elliptical ROI on the reference plane image.

4. The ultrasound system of Claim 2, wherein the plane setting section is further configured to set a plurality of planes that are orthogonal to the reference plane on the volume data, and
wherein the plane image forming section is further configured to form a plurality of plane images corresponding to the plurality of planes based on the volume data.

5. The ultrasound system of Claim 4, wherein the input information further comprises:
third input information for adjusting sizes of the elliptical ROIs set on the plurality of plane images.

6. The ultrasound system of Claim 5, wherein the ROI setting section is further configured to adjust the size of the ellipsoidal ROI based on the third input information to thereby reset the ellipsoidal ROI on the volume data.

7. The ultrasound system of Claim 6, wherein the rendering section is further configured to render volume data corresponding to the reset ellipsoidal ROI to thereby form the 3D ultrasound image.

8. A method of providing a 3D ultrasound image, comprising:
a) acquiring a plurality of ultrasound data for a target object;
b) forming volume data based on the plurality of ultrasound data;
c) setting an elliptical region of interest (ROI) on the volume data based on input information from a user;
d) setting an ellipsoidal ROI on the volume data based on the elliptical ROI; and
e) rendering volume data corresponding to the ellipsoidal ROI to thereby form a 3D ultrasound image.

9. The method of Claim 8, wherein the step c) comprises:
receiving first input information for setting a reference plane on the volume data;
setting the reference plane on the volume data based on the first input information;
forming a reference plane image corresponding to the reference plane based on the volume data;
displaying the reference plane image;
receiving second input information for setting the elliptical ROI on the reference plane image; and
setting the elliptical ROI on the reference plane of the volume data.

10. The method of Claim 8, further comprising:
f) setting a plurality of planes that are orthogonal to the reference plane on the volume data;
g) forming a plurality of plane images corresponding to the plurality of planes based on the volume data;
h) displaying the plurality of plane images.

11. The method of Claim 10, further comprising:
i) receiving third input information for adjusting sizes of the elliptical ROIs set on the plurality of plane images.
j) adjusting the size of the ellipsoidal ROI based on the third input information to thereby reset the ellipsoidal ROI; and
k) rendering volume data corresponding to the reset ellipsoidal ROI to thereby form a 3D ultrasound image.
